# EUROPEAN PATENT APPLICATION

(11) **EP 1 069 181 A2**
(43) Date of publication of application: **17.01.2001**
(21) Application number: 00305590.2
(22) Date of filing: 03.07.2000
(51) Int. Cl.: C12M 1/24, B01L 3/00

(54) **Closure assembly for multiwell vessel**

(30) Priority: 13.07.1999 US 352231; 17.03.2000 EP 00302179
(71) Applicant: Whatman, Inc., Rockland, Massachussetts 02370 (US)
(72) Inventor: Lipsky, Jonathan N, Hanover, Massachusetts 02339 (US); Collins, Ralph W, Green Harbour, Massachusetts 02041 (US); Giacobbe, Robert A, Lavallette, New Jersey 08735 (US)
(74) Representative: Austin, Hedley William

(57) **Abstract**

The closure assembly for a microbiological laboratory vessel (50) having a plurality of wells (96) has a resilient framework (56) including a plurality of sealing elements (58) interconnected by the framework for sealing engagement with at least one of the wells. At least one of the sealing elements includes a passageway (70,71) extending therethrough and filter medium (28) extending across the passageway for allowing sterile exchange of gas therethrough.

## Description

The present invention relates to a closure assembly for a multiwell vessel.

The use of closure devices for covering microbiological vessels, such as flasks, has been a widely accepted and long used practice in microbiology. Closures are used in order to prevent the contamination by airborne contaminates or particulate matter of microorganisms being cultured or stored within the vessels. Such known closures have been used to prevent the airborne escape of microorganisms being cultured or stored in the vessels, to avoid them becoming contaminants themselves.

It is generally an absolute necessity that microorganisms or cultures must be grown under sterile conditions. Likewise, such sterile conditions must be kept in cell cultures and present day genetic manipulations of cells and cell fractions. Depending on the type of microorganism being cultured, either aerobic or anaerobic, closures have been designed to accommodate the specific growth requirements for each of these types of microorganisms. For example, aerobic microorganisms can only live in the presence of oxygen, whereas anaerobic microorganisms are capable of growing in the absence of oxygen, and in some circumstances are unable to grow in the presence of oxygen.

For anaerobic organisms, a closure may be required capable of maintaining sterile conditions within the vessel by preventing the introduction of contaminating microorganisms, while at the same time preventing entry of oxygen into the vessel. The same issues relate to such genetic manipulations as cloning and hybridisation.

Another requirement for a microbiological vessel closure is that, while it should maintain the sterility of the microorganisms or cultures being grown, the closure should provide free access into the vessel to facilitate the addition to the vessel or removal of contents therefrom (for example, by sterile removal of microbiological culture from the vessel).

Historically, cotton or gauze formed into a plug has been inserted into the opening of a vessel of the type described. These cotton or gauze plugs serve the general purpose of preventing contamination of the vessel, while simultaneously permitting free exchange of oxygen with the ambient atmosphere. This type of plug has many deficiencies, in particular it can be difficult to resterilise the plug for subsequent use; furthermore, after repeated usage, the plug tends to readily decompose.

Another similar type of closure is described in US patent 3326401 (De Long), which closure is adapted to fit over the open end of a microorganism container. The closure further includes a disposable plug of a porous material which is positioned within the closure. This device has the deficiency that it does not provide a seal between the closure and the vessel.

Another more recent development in microbiological vessel closures provides the advantage of a filtering device combined with a plug type closure. Such a closure, known as a Steri Plug (CTP Corp. Huntington, New York), is constructed of multiple components including a stopper portion, a filter, and associated gaskets and retainers: Because of its complex design, this type of closure is expensive and cumbersome to use.

A cap for a flask is described in US patent 5180073 with an outer collar and an inner collar, the top portion including a permeable section.

EP-A-0791649 describes and illustrates a closure for sealing a microorganism vessel, which closure includes a resilient seal for sealing the container, a passageway extending through the seal, and a filter medium extending across the passageway integrally moulded to the seal for allowing sterile gas exchange therethrough.

None of the above prior art concern closures for multiwell vessels, suitable for use in microbiological laboratories or the like.

It would be desirable to provide a closure assembly for such a multiwell vessel.

The present invention provides a closure assembly, for sealing a plurality of wells of a multiwell vessel, the closure assembly comprising a resilient framework including a plurality of sealing elements interconnected by the framework for sealing engagement with at least one of the wells into which the respective sealing element is to be disposed, at least one of the sealing elements including a passageway extending therethrough, and filter medium extending across the passageway for allowing substantially sterile gas exchange therethrough.

Preferably, in a closure assembly according to the invention, at least one of the sealing elements is resilient and comprises the passageway and the at least one filter medium, the filter medium being integrally moulded of material different to that of the resilient sealing element. Preferably the filter medium includes a filter membrane and may have support means disposed adjacent thereto for supporting such a filter membrane.

In a preferred embodiment hereinafter more fully described, each of the sealing elements includes a female portion having at least one opening extending therethrough and a male portion having an aperture extending therethrough, the male portion being arranged to be disposed within the female portion with the aperture aligned with a respective opening, the filter medium being contained between the male and female portions and extending across the aperture in the male portion. In a preferred arrangement, each of the male portions includes a shoulder extending outwardly therefrom, so as to form a sealing shoulder completely about a wall of the male portion, for providing a seal against a side wall of the female portion.

In order that the invention may be more clearly understood, there follows a detailed description of exemplary closure members according to the invention and methods for making them. The following detailed description is to be read with reference to the accompanying drawings, in which:
Figure 1 is an exploded perspective view of a preferred embodiment of a closure assembly according to the invention and a multiwell vessel including a plurality of wells;
Figure 2 is a sectional view of portions of the closure assembly and vessel in positions preparatory to forming the closure assembly;
Figure 3 is a sectional view similar to Figure 2 but showing the parts in assembled positions;
Figure 4 is a top view of a further embodiment of the present invention; and
Figure 5 is a side view of the further embodiment of the present invention shown in Figure 4.

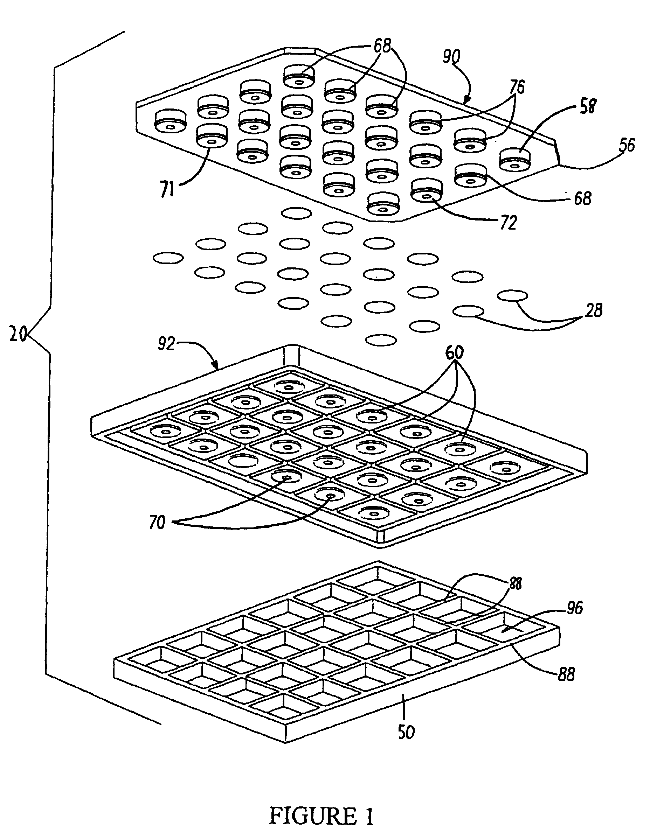

Referring to Figures 1,2 and 3, the combination of a vessel 50 sealed with a closure assembly according to the invention is generally shown and designated by the reference numeral 20. The vessel 50 may contain liquids and microorganisms, cells or cellular components for the purpose of propagating aerobic or anaerobic microorganisms, or conducting other biological manipulations. The vessel 50 is a microorganism container of known multiwell type. The vessel 50 includes a tray having a plurality of wells 96. Referring specifically to Figure 1, the closure assembly comprises a resilient framework 56 which includes a number of sealing plugs 58 which are interconnected by the framework 56.

The resilient framework 56 can be of any suitable material, such as a metal (for example, stainless steel) or plastics.

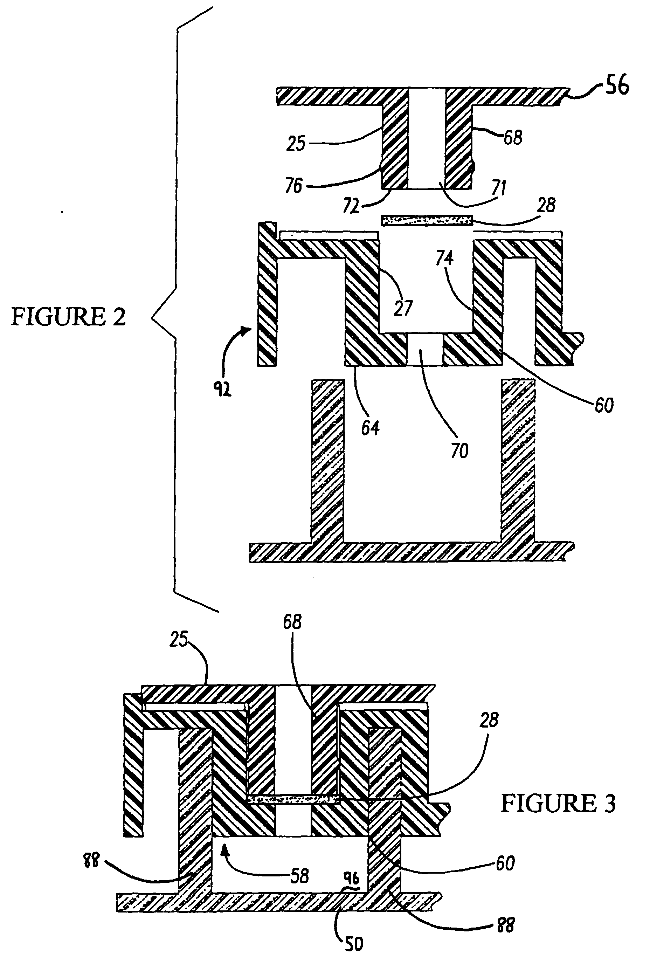

The plugs 58 are preferably of resilient material, capable of sealing by conforming to the contours of the openings of the vessel 50. The plugs 58 therefore deflect and/or yield, so as to sealingly conform to or to sealingly engage with the inner surface wall of the wells 74 (see Figure 2), to form and maintain both an air-tight and liquid-tight seal.

At least an outer surface part of each plug can be of suitable flexible and resilient material, for example, a silicone, a natural or synthetic rubber, a polypropylene or other polyolefin, a polyester, a polyamide, a polystyrene or styrene copolymer, a polycarbonate or a fluoroplast.

As shown in Figures 2 and 3, the combination can be a three part system, the closure assembly including an upper member 90 and a lower member 92. The plug 58 includes a female portion 60 of the lower member 92 having at least one opening 70 extending therethrough and a male portion 68 of the upper member 90, having an aperture 71 extending therethrough. The male portion 68 of the upper member 90 can be disposed within the female portion 60; the opening 70 and aperture 71 provide a passageway extending axially through the aligned male and female portions making up the plug 58.

Trapped between the female portion 60 and the male portion 68 is a filter medium 28 which extends across the opening 70 and aperture 71. The filter medium 28 is such as to permit exchange of gas thereacross, but not the passage of microorganic contaminants. Each filter medium 28 is somewhat flat or disk-shaped and its peripheral edge is sealed between the portions 60 and 68; it extends across the opening 70 and aperture 71 which axially extend through the plug 58. The seal between the peripheral edge and the portions 60 and 59 of plug 58 must be both air-tight and liquid-tight in order to maintain the integrity and/or sterility of the contents of the relevant well sealed by the plug.

The filter medium 28 should be positioned and affixed within the opening through its plug such that fluids (gaseous or liquid) can only pass through the filter medium 28 and not around the periphery of the filter medium 28, which would otherwise breach the sterility of the combination of closure assembly 20 and vessel 50.

The filter medium 28 can include any suitable materials or membranes such as depth media including HEPA or OPA rated glass microfibre, cotton wool, a steel plug, hydrophobic membranes such as polypropylenes, polytetrafluoroethylenes (PTFE), polysulfones, polyvinylidenefluoride (PVDF), or any other suitable porous material. Further, the filter medium may be woven or non-woven and may contain multiple layers. These multiple layers may be made up of the same or different filter medium. The filter medium should, of course, be of a material which is capable of permitting the exchange of gas thereacross, but which will not permit the passage of microorganic contaminants.

The closure assembly 20 can include at least one channel therethrough to allow insertion of tubing, a thermometer or the like therein to be sealed in the channel, for sterile access to the interior of the vessel 50 and the contents without introducing contamination.

The upper member 90 and the lower member 92 can be connected by a bonding technique which is capable of holding the two parts together, for example, glue, heat welding, suction force, ultrasonics or injection moulding.

More specifically, the male portions 68 of the plugs each have a wall 25 extending from the resilient framework 56 thus interconnecting the plugs 58. Each of these plugs 58 has a base portion 72 which includes at least one aperture 71. Additionally, each of the female portions 60 defines a cylindrical cavity 74 having side walls 27 and a bottom base portion 64 which includes an opening 70. Each of the outer walls 25 of the male portion 68 fit in sealing engagement with the side walls 27 of the cavity 74.

Each of the outer walls 25 of the male portions 68 also includes a shoulder 76 extending outwardly therefrom about the outer wall 25, which acts as a sealing ring to engage the side walls 27 of the cavity 74 and further perfect a seal therewith. This sealing engagement thereby forms a liquid-tight seal.

Referring specifically to Figure 1, the multiwell plate comprises the vessel 50 including a plurality of wells 96 which are defined by walls 98. The closure assembly 20 includes the resilient framework 56 which has therein the plurality of seal plugs 58 which are interconnected by the framework 56. The plugs 58 can be forced into sealing engagement with the walls 98 of at least some of the wells 96 into which the plugs 58 are disposed. At least one of the plugs 58 includes a passageway extending therethrough. Additionally, a filter medium 28 extends across the passageway 86 for allowing sterile exchange of gas therethrough in and out of the well 96 upon which the respective plug 58 is disposed.

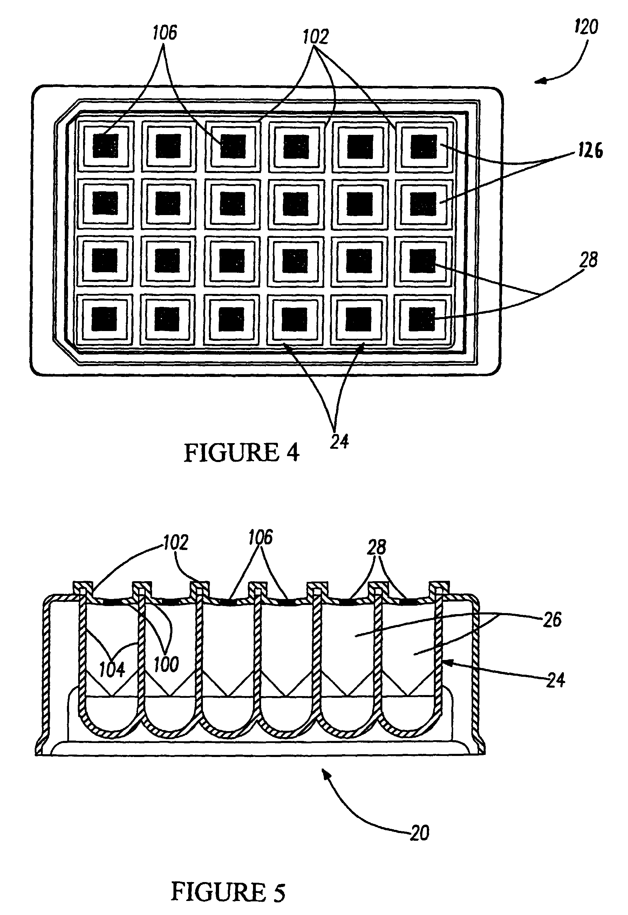

As shown in Figures 4 and 5, a closure assembly 120 may be made of a single piece of material. Preferably, such a closure assembly 120 is injection moulded with the filter medium 28 placed within the assembly 120 such that the peripheral edge 31 of the filter medium 28 is sealed within a passageway 126 in the seal plug 100 while the closure assembly 120 is being formed.

As shown in Figures 4 and 5, the closure assembly can be formed as a single, unitary piece including sealing plug sections 100 surrounded by gripping portions 102. The gripping portions are to sealingly engage the upper portion of walls 104 of a multiwell tray 24. Filter medium 106 is integrally connected to the framework of the plug sections.

The present invention can be practised with various shaped filter media, as long as the latter can be supported and the peripheral edge 31 of the filter medium 28 is available for sealing affixation to the passageway of the sealing plug. Additionally, the present invention can be practised with multiple layers of filter medium 28. These layers may be made up of layers of the same or different filter medium 28. Also, a single sheet of filter medium 28 may be utilised for the entire assembly 20 by placing the filter medium 28 inside the assembly 20 prior to an injection moulding procedure.

The method of forming the closure assembly 20 can include disposing a support for the filter medium within the passageway 26 either during moulding, or subsequent thereto. The method generally includes sizing the filter medium 28 to a desired size. The filter medium 28 and a support therefor can be loaded into a moulded cavity and held in place on top of core pins by locator pins. A suitable material, such as silicone, can then be injected into the mould cavity. The silicone fills the mould cavity and encapsulates the filter medium 28 and support 32, and can then be cured by suitable methods (such as utilizing heat from the mould). After a suitable curing period, the closure assembly 20 can be removed from the mould.

## Claims

1. A closure assembly for sealing a plurality of wells (96) in a multiwell vessel (50), said closure assembly comprising a resilient framework (56) including a plurality of sealing elements (58) interconnected by said framework for sealing engagement with at least one of said wells in which said sealing element is to be located, at least one of said sealing elements including a passageway (70,71) extending therethrough and filter medium (28) extending across said passageway for allowing substantially sterile gas exchange there through.

2. A closure assembly according to claim 1, wherein the passageway of each said sealing element defines a central axis therefor.

3. A closure assembly according to claim 1 or 2, wherein at least one said sealing element is resilient and comprises said passageway and said at least one said filter medium, said filter medium being integrally moulded of material different to that of said resilient sealing element.

4. A closure assembly according to any of claims 1 to 3, wherein said filter medium includes a filter membrane (28).

5. A closure assembly according to claim 4, wherein said filter membrane (28) includes at least one support means disposed adjacent to said filter membrane for supporting said filter membrane.

6. A closure assembly according to any of claims 1 to 5, wherein each said sealing element comprises a female portion (60) having at least one opening (70) extending therethrough and a male portion (68) including an aperture (71) extending therethrough, said male portion being arranged to be disposed within said female portion with said aperture aligned with a respective opening, said filter medium being contained between said male and female portions and extending across said aperture.

7. A closure assembly according to claim 6, wherein each said male portion includes a shoulder (76) extending outwardly therefrom so as to form a sealing shoulder completely about said male portion for perfecting a seal against the respective side wall (27) of said female portion.

8. A closure assembly according to any of the preceding claims, wherein at least one of said sealing elements includes at least one channel extending therethrough to allow insertion of tubing therethrough.

9. A multiwell vessel apparatus for use in microbiological laboratories or the like, the apparatus comprising a tray including a plurality of wells therein, a closure assembly according to any one of claims 1 to 8 arranged to seal at least one of said wells.
